(19)

Europäisches
Patentamt
European
Patent Office
Office européen
des brevets

(11) **EP 4 509 837 A1**

(12) **EUROPEAN PATENT APPLICATION**

(43) Date of publication:
**19.02.2025 Bulletin 2025/08**

(21) Application number: **23197774.5**

(22) Date of filing: **15.09.2023**

(51) International Patent Classification (IPC):
**G01N 33/558** (2006.01)   **G01N 33/82** (2006.01)

(52) Cooperative Patent Classification (CPC):
**G01N 33/558; G01N 33/82**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.08.2023 CN 202311044948**

(71) Applicant: **Zhejiang Wantaifu Biotechnology Co.,
Ltd
Hangzou Zhejiang (CN)**

(72) Inventors:
• **CAI, Jiangtao
Zhejiang (CN)**
• **GUAN, Hongyan
Zhejiang (CN)**
• **HE, Qing
Zhejiang (CN)**

(74) Representative: **Gilani, Anwar et al
Venner Shipley LLP
406 Science Park
Milton Road
Cambridge CB4 0WW (GB)**

(54) **KIT FOR DETECTING 25-HYDROXYVITAMIN D IN SAMPLE AND DETECTION METHOD THEREOF**

(57)    The present invention provides a detection kit for detecting 25-hydroxyvitamin D and a detection method thereof. Formulations of a dissociating agent and an antibody dry powder are known through screening and optimization, such that self-mixing and dissociation of a sample are realized without being affected by human factors and depending on any mixing equipment, ensuring a 25-hydroxyvitamin D antibody is fully reacted with a substance to be tested. In addition, an operating process of an immunochromatography assay is improved, such that dissociation of the sample and bonding of the 25-hydroxyvitamin D antibody and the substance to be tested are completed in one reaction tube and completely separated from a test strip; and the test strip is employed for sample injection and detection after completion of reaction, and a specific control line is arranged. This significantly improves the accuracy and sensitivity of portable detection of 25-hydroxyvitamin D with the immunochromatography assay, and the detection sensitivity of the 25-hydroxyvitamin D reaches 1 ng/mL.

**EP 4 509 837 A1**

**Description**

CROSS-REFERENCE TO RELATED APPLICATIONS

This application claims priority to Chinese Patent Application, Application No.: 2023110449482, filed on August 17, 2023, and the specification, claims, abstract, and accompanying drawings of this application are incorporated by reference in their entirety as a part of this application.

BACKGROUND OF THE INVENTION

Field of the Invention

The present invention relates to the technical field of detection of 25-hydroxyvitamin D, and in particular, to a dissociation and detection kit for 25-hydroxyvitamin D and a detection method thereof.

Description of the Related Art

Serum 25-hydroxyvitamin D is the main form of vitamin D in the body. Vitamin D is an essential nutritional component for the growth, breeding, life sustaining and health maintenance of humans, livestock and poultry. Vitamin D obtained from both dietary and dermal routes, in conjunction with plasma $\alpha$-globulin, is transported to the liver, and then transformed into 25-hydroxyvitamin D in the endoplasmic reticula and mitochondria of hepatocytes under the action of 25-hydroxylase. The content of serum 25-hydroxyvitamin D reflects the content of vitamin D in the body and correlates with clinical symptoms of vitamin D deficiency.

At present, common detection methods for 25-hydroxyvitamin D include chemiluminescent immunoassay, enzyme linked immunosorbent assay, chemiluminescence immunoassay, and the like. Most of the methods are time-consuming and cumbersome, and require utilization of large-scale instruments. This is not conducive to "generalization" of testing. In contrast, an immunochromatography assay is easy and quick to operate and does not require additional instruments. It can realize primary detection and therefore is conveniently used in primary hospitals, clinics and remote areas. In the present invention, the detection kit has the advantages of portability, simple operation, high coincidence rate, and strong stability, and is suitable for home monitoring, primary clinics and medical institutions without large instruments.

However, detection of 25-hydroxyvitamin D with the immunochromatography assay in the current market has the following problems:

1) During the dissociation of 25-hydroxyvitamin D in a sample, a mixing effect will directly affect a dissociation effect. In existing methods, it is often necessary to fully mix a dissociation solution with a sample to be tested by hand or equipment; mixing by hand causes the mixing and dissociation effect to be greatly influenced by human factors; mixing by equipment requires large-scale vibration and ultrasound equipment and the like, which is inconvenient to carry out. Therefore, it is also necessary to find a way to self-mix samples without depending on mixing by hand or equipment.

For example, in the patent CN 115166266 A, a dissociating agent has a complex formulation, and a way to mix a solution A and a solution B in proportion cannot be used to self-mix the dissociating agent and terminate dissociation thereof. The existence of the dissociation solution will have an impact on subsequent reaction, which is greatly influenced by human factors.

2) The antigen-antibody reaction time of the immunochromatography assay is relatively short. In existing methods, a 25-hydroxyvitamin D antibody is usually immobilized in a test strip, and reacts with 25-hydroxyvitamin D in a liquid-phase sample through a solid-phase antibody; and there is a problem of non-uniformity of solid-phase and liquid-phase reaction, resulting in insufficient reaction of the 25-hydroxyvitamin D antibody with the 25-hydroxyvitamin D in the sample, thereby affecting the detection sensitivity and gradient.

For example, in the patent CN115856326A, although the test strip prolongs an antigen-antibody reaction channel and increases an antigen-antibody reaction time due to the existence of a specially treated connecting bridge, it still belongs to reaction of the solid-phase antibody with the liquid-phase sample. Such reaction is insufficient and easily affected by a sample size (liquid phase). When the sample size is excessively large, the antigen-antibody reaction time is not ideal due to the influence of chromatography, thereby reducing the detection sensitivity.

A detection kit for 25-OH-VD is provided in CN115932289A. A probe freeze-dried powder and a sample are together added into a sample treatment solution and mixed well, and then added into a test strip. However, such mixing cannot be performed at will, and the manual mixing time thereof is relatively short (30s), so it is difficult to ensure the uniformity of the mixing effect. Moreover, the probe freeze-dried powder also includes control antibodies. Due to the existence of various antibodies, a reaction process is more complex and has more interference, thereby affecting the detection sensitivity and accuracy. Although the detection sensitivity can reach 5 ng/mg, it is greatly affected by human factors because the mixture needs to be shaken manually.

Therefore, it is urgent to find a novel dissociation and detection method for 25-hydroxyvitamin D. With the use of the dissociation method, dissociation of 25-hydroxyvitamin D in the sample is not affected by human factors, and the 25-hydroxyvitamin D is mixed and dissociated at will. At the same time, the 25-hydroxyvitamin D antibody can be allowed to fully react with the 25-hydroxyvitamin D in the sample, thereby ensuring the accuracy and sensitivity of test results.

BRIEF SUMMARY OF THE INVENTION

To resolve problems existing in the prior art, the present invention provides a dissociation and detection kit for 25-hydroxyvitamin D and a detection method thereof. Formulations of a dissociating agent and an antibody dry powder are known through screening and optimization, such that self-mixing and dissociation of a sample are realized without being affected by human factors and depending on any mixing equipment, ensuring a 25-hydroxyvitamin D antibody is fully reacted with a substance to be tested. In addition, an operating process of an immunochromatography assay is improved, such that dissociation of the sample and bonding of the 25-hydroxyvitamin D antibody and the substance to be tested are completed in one reaction tube and completely separated from a test strip; and the test strip is employed for sample injection and detection after completion of reaction, and a specific control line is arranged. This significantly improves the accuracy and sensitivity of portable detection of 25-hydroxyvitamin D with the immunochromatography assay.

In one aspect, the present invention provides a dissociating agent of 25-hydroxyvitamin D, and the dissociating agent includes a protein decomposer, a surfactant, a detergent, and a metal complexing agent; the protein decomposer includes guanidinium salt and strong acid; the surfactant includes triton X-100 and a surfactant S9; the detergent includes sodium dodecyl sulfate; and the metal complexing agent includes ethylene diamine tetraacetic acid.

Further, the strong acid is citric acid, and the guanidinium salt is guanidine hydrochloride.

In the present invention, the dissociating agent of 25-hydroxyvitamin D is screened for composition and formulation, such that the dissociating agent is easily self-mixed well during the dissociation thereof, and the dissociating effect thereof is more sufficient and is not affected by human factors. Therefore, this helps improve the detection accuracy and sensitivity of the 25-hydroxyvitamin D.

The guanidinium salt and the citric acid in the dissociating agent used in the present invention destroy protein to release the 25-hydroxyvitamin D; the surfactant is the triton X-100 and the surfactant S9, such that reaction of dissociated free vitamin D with a subsequent vitamin D antibody is more specific; the detergent is the sodium dodecyl sulfate to allow substances in the reaction tube including freeze-dried/dried powder to be fully dissolved and reacted; the metal complexing agent is the ethylene diamine tetraacetic acid to avoid interference of metal ions on immune reaction; the citric acid can not only destroy the protein to release the 25-hydroxyvitamin D, but also react with sodium carbonate in the reaction tube to generate gas to cause the solution to be self-mixed well, such that the dissociated free 25-hydroxyvitamin D can more fully react with a subsequent colloidal gold-labeled 25-hydroxyvitamin D antibody, improving the detection sensitivity.

Further, each 1 L of the dissociating agent includes 1-20 g of guanidine hydrochloride, 1-20 mL of triton X-100, 1-20 g of sodium dodecyl sulfate, 1-20 g of ethylene diamine tetraacetic acid, 50-400 g of citric acid, and 1-20 g of surfactant S9; and the remainder thereof is water.

In another aspect, the present invention provides a 25-hydroxyvitamin D antibody powdered reagent, and the powdered reagent includes an anti-25-hydroxyvitamin D antibody, an antibody protective agent, an antibody fixative, and sodium carbonate; the antibody fixative includes polyvinyl alcohol and polyvinylpyrrolidone; and the antibody is conjugated with a label. The label described herein may be color particles such as gold particles, latex particles, a water-soluble dye, a fluorescent substance, and a radioactive substance.

Further, the antibody protective agent includes casein sodium salt and trehalose.

A special antibody dry powder reaction tube is designed in the present invention, such that dissociation of the sample and reaction of dissociated antigens and antibodies are simultaneously completed in the antibody dry powder reaction tube; the antigens and the antibodies are completely separated from the test strip, such that they are more thoroughly dissociated and more fully and effectively react. The test strip is employed for sample injection and detection after completion of reaction. This significantly improves the accuracy and sensitivity of portable detection of 25-hydroxyvitamin D with the immunochromatography assay.

In the present invention, the colloidal gold-labeled 25-hydroxyvitamin D antibody is freeze-dried or dried in the antibody dry powder reaction tube, and adhered to the reaction tube through polyvinyl alcohol and polyvinylpyrrolidone to avoid the insufficient dosage of the antibody during the reaction due to falling off thereof during transportation, which further affects the color development of subsequent detection cards. The casein sodium salt and the trehalosecan can effectively protect the colloidal gold-labeled 25-hydroxyvitamin D antibody. The sodium carbonate is used to adjust PH and react with the citric acid in the dissociating agent to generate gas, such that the solution can be self-mixed well. The sodium carbonate can be completely dissolved about 5-10 min, and the dissociation of the 25-hydroxyvitamin D in the protein is terminated. At the same time, the PH of the solution becomes neutral, without affecting the next antigen-antibody immune reaction.

Further, the powdered reagent includes 0.8-8 $\mu$g of a colloidal gold-labeled murine anti-25-hydroxyvitamin D monoclonal antibody, 0.008-0.8 μg of casein sodium salt, 0.008-0.8 μg of polyvinyl alcohol, 0.008-0.8 μg of polyvinyl-pyrrolidone, 400-4000 μg of sodium carbonate, and 0.8-8 μg of trehalose.

In yet another aspect, the present invention provides a detection kit for 25-hydroxyvitamin D; the kit includes the dissociating agent, a 25-hydroxyvitamin D antibody dry powder reaction tube, and a 25-hydroxyvitamin D immunochromatography detection card; and the 25-hydroxyvitamin D antibody dry powder reaction tube is filled with the 25-hydroxyvitamin D antibody dry powder.

Further, the 25-hydroxyvitamin D immunochromatography detection card includes the test strip, and a sample application area, a reaction area and a water absorption area that are sequentially connected with the test strip; the reaction area is provided with a test line and a control line; a 25-hydroxyvitamin D complete antigen is coated on the test line; and a colloidal gold-labeled murine IgG antibody and 0.005%-0.5% Evans blue are coated on the control line.

In the test strip provided in the present invention, the control line is given a new design and fixed with two layers of substances, where the colloidal gold-labeled murine IgG antibody (red) is coated on the lower layer thereof, and Evans blue (blue) is located on the upper layer thereof The Evans blue on the upper layer thereof completely covers the color of colloidal gold. Therefore, the control line is blue in an initial state when not tested.

When the sample is correctly sampled, the excessive sample will wash away the Evans blue; after the blue is washed away, the next layer of red will be displayed, indicating valid test; however, when an amount of the sample added into the detection card is too low to wash away the Evans blue on the control line, the control line is still displayed in blue, indicating invalid test.

In existing control lines, it is usually necessary to place an antigen that can bind to the antibody coated on the control line in advance into the reaction liquid or on the test strip, and the addition of the antigen also affects the detection sensitivity of 25-hydroxyvitamin D.

In the design of the control line provided in the present invention, it is not necessary to introduce other antigens into the reaction liquid or the test strip for control detection. This can reduce the influence on the detection sensitivity of 25-hydroxyvitamin D and facilitate a user to observe more intuitively whether an enough amount of the sample is added, better avoiding the influence on test results due to a low amount of the sample added, thereby ensuring the accuracy of the test results.

In yet another aspect, the present invention provides a dissociation method for 25-hydroxyvitamin D. In the dissociation method, the dissociating agent is used for dissociation.

In yet another aspect, the present invention provides a detection method for 25-hydroxyvitamin D. In the detection method, the kit is used for detection, and the detection method includes the following steps:

(1) adding a sample to be tested and a dissociating agent into a 25-hydroxyvitamin D antibody dry powder reaction tube, dissociating the sample, and conducting labeling reaction for 5-10 min to obtain a reaction liquid; and
(2) adding the reaction liquid into a sample application well of a 25-hydroxyvitamin D immunochromatography detection card.

The present invention has the following beneficial effects:

1. Dissociation of the sample and bonding of the 25-hydroxyvitamin D antibody and the substance to be tested are completed in one reaction tube and completely separated from a test strip; and the test strip is employed for sample injection and detection after completion of reaction. This significantly improves the accuracy and sensitivity of portable detection of 25-hydroxyvitamin D with the immunochromatography assay.
2. After the dissociating agent is added into the antibody dry powder reaction tube, self-mixing of the sample can be realized without being affected by human factors and depending on any mixing equipment.
3. The composition and formulation of the dissociating agent are familiarized through screening, such that the dissociating agent is more easily self-mixed during the dissociation, and the dissociating effect thereof is more sufficient and effective. This helps improve the detection accuracy and sensitivity of the 25-hydroxyvitamin D.
4. The composition and formulation of an antibody dry powder are familiarized through screening, such that the antigen-antibody reaction is also more sufficient and effective. This helps improve the detection accuracy and sensitivity of the 25-hydroxyvitamin D.
5. The detection sensitivity of the 25-hydroxyvitamin D reaches 1 ng/mL.

BRIEF DESCRIPTION OF THE DRAWINGS

FIG. 1 is a structural schematic diagram of a test strip provided in Embodiment 1;
FIG. 2 is a structural schematic diagram of an immunochromatography detection card provided in Embodiment 1;

FIG. 3 is a schematic diagram of a detection process for 25-hydroxyvitamin D provided in Embodiment 1; and

FIG. 4 is a standard colourimetric card provided in Embodiment 1.

## DETAILED DESCRIPTION OF THE INVENTION

The following further describes the structures involved in the present invention or the technical terms used therein. Unless otherwise specified, they shall be understood and explained according to the general terms commonly used in the prior art.

Detection

Detection means assaying or testing presence or absence of a substance or material, including but not limited to, chemical substance, organic compound, inorganic compound, metabolite, drug, drug metabolite, organic tissue, metabolite of organic tissue, nucleic acid, protein or polymer. In addition, detection means that the amount of a substance or material is tested. Further, assay also means immunoassay, chemical assay, enzyme assay, and the like.

Sample

The samples detected by the test device of the present invention include biological liquid (for example, case liquid or clinical sample). Liquid samples or liquid specimens may be derived from solid or semi-solid samples, including feces, biological tissues and food samples. The solid or semi-solid samples may be converted to liquid samples by any appropriate methods, such as mixing, mashing, macerating, incubating, dissolving, or digesting the solid samples by enzymolysis in suitable solutions, such as water, phosphate solutions, or other buffer solutions. "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological sample is urine, and preferably, the biological sample is saliva. Food samples include food processed materials, final products, meat, cheese, wine, milk, and drinking water. Plant samples include samples derived from any plants, plant tissues, plant cell cultures, and media. "Environmental samples" include samples derived from the environment (e.g., liquid samples from lakes or other bodies of water, sewage samples, earthen samples, groundwater, seawater, and waste liquid samples). The environmental sample may further include sewage or other waste water.

An appropriate test device according to the present invention can be used to detect any analyte. Preferably, the test device of the present invention is used to detect small drug molecules in saliva and urine. Of course, the samples detected by the test device of the present invention may be any samples of the above forms, regardless of being solid or liquid at the beginning, provided that these liquids or liquid samples can be absorbed by the sample application area of the testing element. Generally, the sample application area is made of a water absorbent material, and liquid samples or liquid specimens can be absorbed by the capillary or other characteristics of the material of an absorption element, such that the liquid sample can flow in the sample application area. The material of the liquid sample application area may be any material capable of absorbing liquid, such as sponge, filter paper, polyester fiber, gel, non-woven fabric, cotton, polyester film, and yarn. Of course, the liquid sample application area may be made of a water absorbent material or a non-water absorbent material. However, the absorption element is provided with holes, screw threads, and caves on which the samples can be collected. Generally, the samples are solid or semi-solid samples, and filled between screw threads and in the holes or caves for collection. Of course, optionally, the sample application area may be composed of some non-absorbent fibers and hairs, and these materials are used to scrape a solid, semi-solid or liquid sample, such that these samples can be retained on the sample application area.

Downstream and upstream

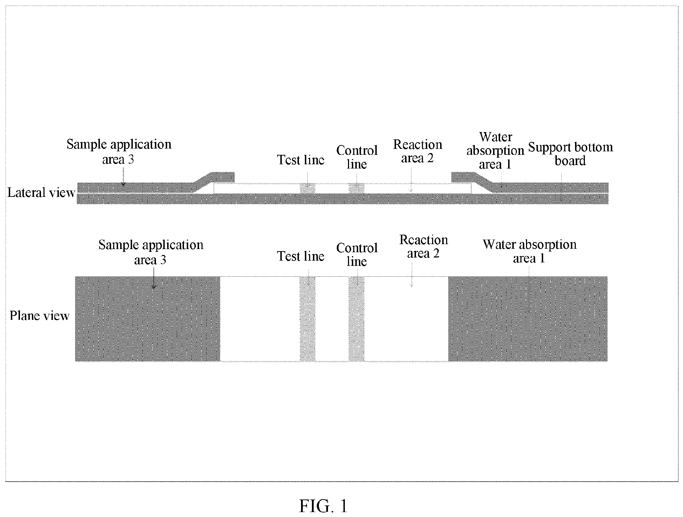

Downstream or upstream is divided according to a flow direction of a liquid, generally, a liquid or fluid flows to a downstream area from an upstream area. The downstream area receives the liquid from the upstream area, and a liquid also may flow to a downstream area along an upstream area. Here, downstream or upstream is generally divided according to a flow direction of a liquid, for example, on some materials where capillary force is utilized to promote the flow of a liquid, a liquid may overcome gravity to flow towards an opposite direction to the gravity; and in this case, downstream or upstream is divided according to a flow direction of the liquid. For example, in the test device of the present invention, after a diversion element receives the liquid sample, fluid can flow from the diversion element to a sample application area or a sample application pad of two testing elements, and then liquid flowing to the sample application pad flows to a downstream label pad and is mixed with the marked label; and the mixture flows to a downstream testing pad through a transition pad, where a testing area on the testing pad is located upstream of a test result control area, such that the mixture finally flows to an absorption pad on a downstream absorption area. The testing area may be a polyester fiber film, and the diversion element may be a glass fiber, a polyester chip, and a polyester film. In this case, the diversion element is located at the upstream of the label area of the testing element. The specific structure of the testing element is a structure 20 as shown in FIG. 1 and FIG. -2. Liquid on a part of the sample application pad flows mainly by a capillary force.

Gas flow or liquid flow

Gas flow or liquid flow means that liquid or gas can flow from one place to another place. In a flow process, the liquid or gas may pass through some physical structures to play a guiding role. The "passing through some physical structures" here means that liquid passes through the surface of these physical structures or their internal space and flows to another place passively or actively, where passivity is usually caused by external forces, such as flow under the capillary action and the action of air pressure. The flow here may also be a flow due to self-action (gravity or pressure) of the liquid or gas, and also may be a passive flow. The fluid under the action of air pressure may be a forward flow, or also a reverse flow; or a fluid is urged to flow to another position from a position under the action of air pressure. Here, the flow does not mean that a liquid or a gas is necessarily present, but indicates a relationship or state between two objects under some circumstances. In case of presence of liquid, it can flow from one object to another. Here it means the state in which two objects are connected. In contrast, if there is no gas flow or liquid flow state between two objects, and liquid exists in or above one object but is unable to flow into or on another object, it is a non-flow, non-liquid or non-gas flow state.

Testing element

The "testing element" used herein refers to an element that can be used to detect whether a fluid sample or a fluid specimen (a liquid sample or a liquid specimen) contains an interested analyte. Such testing can be based on any technical principles, such as immunology, chemistry, electricity, optics, molecular science, nucleic acids, and physics. The testing element can be a lateral flow test strip that can detect a variety of analytes. Of course, other suitable testing elements can also be used in the present invention. In the present invention, the testing element and the "lateral flow testing element, or test strip" can be used interchangeably, indicating same meanings.

Various testing elements can be combined for use in the present invention. One form of the testing elements is a test strip. The test strips used for analyzing the analyte (such as drugs or metabolites that show physical conditions) in samples can be of various forms such as immunoassay or chemical analysis. The analysis mode of non-competition law or competition law can be applied for test strips. A test strip generally contains a water absorbent material that has a sample application area, a reagent area, and a testing area. Fluid or liquid samples are added to the sample application area and flow to the reagent area under the capillary action. If analyte exists in the reagent area, samples will bind to the reagent. Then, samples continue to flow to the testing area. Other reagents such as molecules that specifically bind to analyte are immobilized on the testing area. These reagents react with the analyte (if any) in the sample and bind to the analyte in this area, or bind to a reagent in the reagent area. Label used to display the detection signal exists in the reagent area or the detached label area.

Typical non-competition law analysis mode: if a sample contains analyte, a signal will be generated; and if not, no signal will be generated. Competition law: if no analyte exists in the sample, a signal will be generated; and if analyte exists, no signal will be generated.

The testing element can be a test strip, which can be water absorbent material or non-water absorbent material. The test strip can contain several materials used for delivery of liquid samples. One material of the test strip can cover the other material thereof. For example, the filter paper covers the nitrocellulose membrane. One or more materials may be used in one area of the test strip, and one or more other different materials may be used in the other area thereof. The test strip can stick to a certain support or on a hard surface for improving the strength of holding the test strip.

Analyte is detected through a signal generating system. For example, one or more enzymes that specifically react with this analyte is or are used, and the above method of fixing a specific binding substance on the test strip is used to fix the combination of one or more signal generating systems in the analyte testing area of the test strip. The substance that generates a signal can be in the sample application area, the reagent area or the testing area, or on the entire test strip, and one or more materials of the test strip can be filled with this substance. The solution containing a signifier is added onto the surface of the test strip, or one or more materials of the test strip is or are immersed in a signifier-containing solution. The test strip containing the signifier solution is made dry.

Various areas of the test paper can be disposed as follows: sample application area, label, and testing area, where the testing area includes a test result area and a test result control area. The control area is located behind or downstream of the testing area. All areas can be disposed on a test strip that is only made of one material. Alternatively, different areas may be made of different materials. Each area can be in direct contact with the liquid sample, or different areas are arranged according to the flow direction of liquid sample; and a tail end of each area is connected and in overlapped with the front end of the other area. Materials used can be those with good water absorption such as filter papers, glass fibers or nitrocellulose membranes. The test strip can also be in other forms.

The nitrocellulose membrane test strip is commonly used, that is, the testing area includes a nitrocellulose membrane (NC) on which a specific binding molecule is immobilized to display the test result; and other test strips such as cellulose acetate membrane or nylon membrane test strips can also be used. For example, test strips and similar devices with test strips disclosed in the following patents: US 4857453; US 5073484; US 5119831; US 5185127; US 5275785; US 5416000; US 5504013; US 5602040; US 5622871; US 5654162; US 5656503; US 5686315; US 5766961; US 5770460; US 5916815; US 5976895; US 6248598; US 6140136; US 6187269; US 6187598; US 6228660; US 6235241; US 6306642; US 6352862; US 6372515; US 6379620, and US 6403383. The test strips and similar device with test strips disclosed in the above patents may be applied to the testing element or test device of the present invention for the detection of an analyte, for example, the detection of an analyte in a sample.

Test strips used in the present invention may be commonly referred as lateral flow test strips. The specific structure and detection principle of the test strips are well known to a person skilled in the art in the prior art. A common test strip includes a sample collection area or a sample application area, and a testing area.

Analyte

Examples where an analyte related to the present invention can be used include some small-molecule substances, including drugs (such as drug of abuse). "Drug of Abuse" (DOA) refers to using a drug (playing a role of paralyzing the nerves usually) not directed to a medical purpose. Abuse of these drugs will lead to physical and mental damage, dependency, addiction and/or death. Examples of drug abuse include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzodiazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The test device of the present invention may also be used for detecting drugs belonging to a medical use but easy to be taken excessively, such as tricyclic antidepressants (imipramine or analogues) and acetaminophen. These drugs are metabolized into micromolecular substances after absorbed by human body. These micromolecular substances exist in blood, urine, saliva, sweat and other body fluids or in some body fluids.

For example, the analyte detected by the pre invention includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis, Camyplobacter genus, L. monocytogenes, Vibrio,* or *Bacillus cereus*) and substances related with physiological features in a urine sample, such as pH and specific gravity. Chemical analysis of any other clinical urine may be performed by lateral flow test in combination with the device of the present invention. Such chemical analysis can be also used to detect the presence of virus antigens, such as COVID-19 antigen and influenza antigen.

Embodiments

The following further elaborates preferred embodiments of the present invention with reference to the accompanying drawings, and it should be noted that the embodiments described hereinafter are intended to facilitate the understanding of the present invention and do not impose any restriction on it.

**Embodiment 1: Detection kit for 25-hydroxyvitamin D and detection method thereof provided in the present invention**

I. Preparation of detection kit for 25-hydroxyvitamin D

The detection kit for 25-hydroxyvitamin D provided in the present invention includes the following parts:

1. Preparation of dissociating agent

Weigh 5 g of guanidine hydrochloride, 1 mL of triton X-100, 1 g of sodium dodecyl sulfate, 1 g of ethylene diamine tetraacetic acid, 100 g of citric acid, 1 g of surfactant S9, and ultra-pure water as the remainder to prepare 1 L of the dissociating agent.

2. Provision of colloidal gold-conjugated labeled antibody dry powder reaction tube

Preparation of 10 $\mu$L of reaction liquid: weigh 0.8 $\mu$g of colloidal gold-labeled murine anti-25-hydroxyvitamin D monoclonal antibody (purchased from COMBIO, model: 2-D3715M), 0.008 $\mu$g of casein sodium salt, 0.008 $\mu$g of polyvinyl alcohol, 0.008 $\mu$g of polyvinylpyrrolidone, 400 $\mu$g of sodium carbonate, 0.8 $\mu$g of trehalose, and ultra-pure water as the remainder.

Add 10 $\mu$L of reaction liquid into an extraction tube, and then perform drying at 37°C to obtain a red solid powder adhered to a tube wall. In such a way, an antibody dry powder reaction tube is prepared.

3. 25-hydroxyvitamin D immunochromatography detection card

A test strip is placed in the detection card. As shown in FIG. 1, the test strip includes a water absorption area 1, a reaction area 2, and a sample application area 3. The preparation method of the water absorption zone 1 is that 18mm absorbent paper is dried in a 50°C oven for later use.

The preparation method of the reaction area 2 is that a 25-hydroxyvitamin D antigen is diluted to a specified concentration with a coating diluent (PBS phosphate buffer) for later use. The murine IgG antibody is labeled with colloidal gold, added with 0.05% Evans blue, and diluted to the specified concentration with the coating diluent for later use.

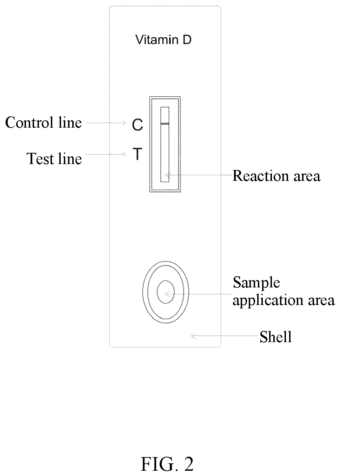

The reaction area is provided with a test line and a control line, and the 25-hydroxyvitamin D antigen (purchased from COMBIO, model: 2-D3726H) is immobilized on the test line; a colloidal gold-labeled murine IgG antibody (purchased from Fuzhou Chuangdian Biology Technology Co., Ltd., model: CDQT-101) and 0.05% Evans blue are coated on the control line. The colloidal gold-labeled murine IgG antibody is located on the lower layer thereof, and the Evans blue on the upper layer thereof completely covers the color of colloidal gold, so the control line is blue in an initial state. Generally, the colloidal gold-labeled murine IgG antibody is mechanically immobilized on the control line, and then an Evans blue line is drawn thereon to cover a colloidal gold-labeled murine IgG antibody line. The structural schematic diagram of the detection card is as shown in FIG. 2.

II. Detection method

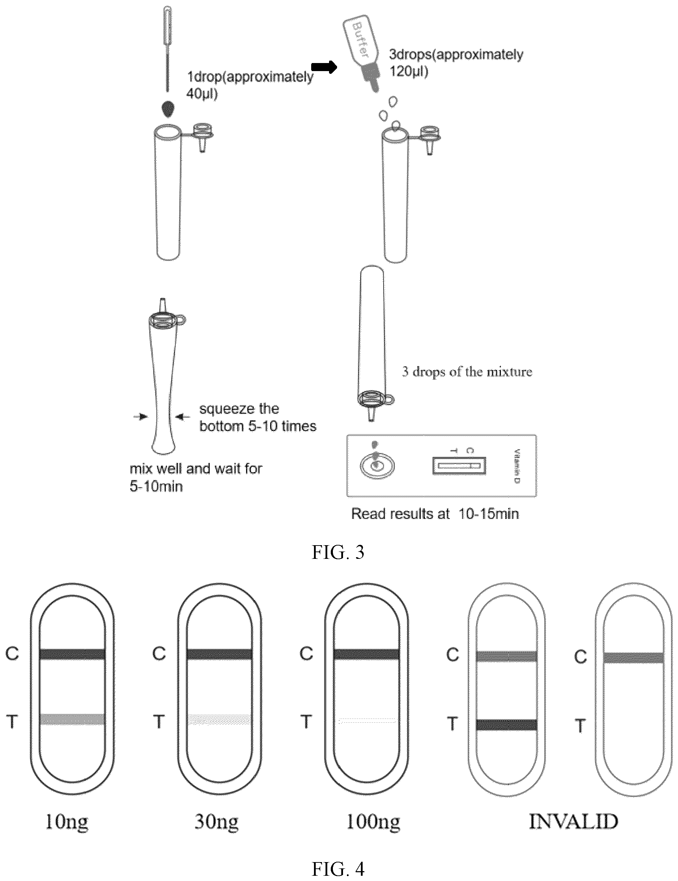

A method for detecting 25-hydroxyvitamin D in a blood sample in vitro by a detection kit for 25-hydroxyvitamin D provided in the embodiment, as shown in FIG. 3, specifically includes:

   1) Take 40 $\mu$L of a sample to be tested (serum/plasma/whole blood/peripheral blood) in a reaction tube, add 120 $\mu$L of a dissociating agent, shake the mixture well, cover an extraction tube, and react the mixture for 5-10 min (10 min is preferred in the embodiment) at ambient temperature;
   2) Add 3 drops of a reaction liquid into a sample application well 4 of a 25-hydroxyvitamin D immunochromatography detection card, and perform reaction for 10-15 min at ambient temperature; and
   3) Directly read test results (suitable for outdoor portable detection), and detect the fluorescence intensity (fluorescent dyes rather than gold particles are labeled) with a fluorescence immunoassay analyzer to calculate the content of the 25-hydroxyvitamin D.

III. Detection principle

Because the antibody dry powder reaction tube includes the colloidal gold-labeled murine anti-25-hydroxyvitamin D monoclonal antibody, after the sample to be tested and the dissociating agent are together added into the antibody dry powder reaction tube, the 25-hydroxyvitamin D antigen in the sample to be tested is bonded with the colloidal gold-labeled murine anti-25-hydroxyvitamin D monoclonal antibody in the antibody dry powder reaction tube. Because there is an excessive amount of the colloidal gold-labeled murine anti-25-hydroxyvitamin D monoclonal antibody in the antibody dry powder reaction tube, a part of the colloidal gold-labeled murine anti-25-hydroxyvitamin D monoclonal antibody is still unbound. When the reaction liquid is added into the detection card, the remaining colloidal gold-labeled murine anti-25-hydroxyvitamin D monoclonal antibody unbound is captured by the 25-hydroxyvitamin D antigen coated on the test line (T line), thereby achieving color development. The 25-hydroxyvitamin D antigen in the sample to be tested is in a competitive relationship with the 25-hydroxyvitamin D antigen coated on the test line. When the content of the 25-hydroxyvitamin D antigen in the sample to be tested is high, an amount of the remaining colloidal gold-labeled murine anti-25-hydroxyvitamin D monoclonal antibody unbound is low and an amount of the colloidal gold-labeled murine anti-25-hydroxyvitamin D monoclonal antibody bound with the 25-hydroxyvitamin D antigen coated on the test line is also low, with development of light color. When the content of the 25-hydroxyvitamin D antigen in the sample to be tested is low, the amount of the remaining colloidal gold-labeled murine anti-25-hydroxyvitamin D monoclonal antibody unbound is large and the amount of the colloidal gold-labeled murine anti-25-hydroxyvitamin D monoclonal antibody bound with the 25-hydroxyvitamin D antigen coated on the test line is also large, with development of deep color.

Therefore, for the detection card provided in the present invention, the low content of the 25-hydroxyvitamin D in a corresponding sample indicates high color development, and therefore the detection card is more conducive to the detection of the content of low-concentration 25-hydroxyvitamin D; and the fluorescence immunoassay analyzer can be used to analyze a specific fluorescence intensity and has relatively high detection sensitivity. In addition, the content of 25-hydroxyvitamin D in blood is usually low and is generally within the range of 30-100 ng/ml under normal circumstances. However, when the 25-hydroxyvitamin D is insufficient, the content thereof may drop to a very low level, and the detection card provided in the present invention may be suitable for 1 ng/ml-80 μg/ml.

The control line is displayed in blue when not tested, and the red control line indicates an effective test. When the sample is correctly sampled, the excessive sample will wash away the Evans blue; after the blue is washed away, the next layer of red will be displayed, indicating valid test; however, when an amount of the sample added into the detection card is too low to wash away the Evans blue on the control line, the control line is still displayed in blue, indicating invalid test.

IV. Analysis of test results

1. Reading test results on the detection card directly

According to a standard colourimetric card as shown in FIG. 4, the content of the 25-hydroxyvitamin D in the sample can be directly read as 0 ng/mL, 10 ng/mL, 30 ng/mL, or 100 ng/mL.

2. Detection by fluorescence immunoassay analyzer

1) Preparation of standard sample

A 25-hydroxyvitamin D standard is prepared into a standard solution and a control with negative blank human serum; and the 25-hydroxyvitamin D standard has eight series of concentrations (S1-S8), as shown in Table 1:

Table 1 Eight series of concentrations of the standard

| Series | 25-hydroxyvitamin D (ng/mL) |
|--------|------------------------------|
| S1 | 1 |
| S2 | 3 |
| S3 | 5 |
| S4 | 10 |
| S5 | 30 |
| S6 | 100 |
| S7 | 500 |
| S8 | 1000 |

The control has three series of concentrations of low (L), medium (M), and high (H), as shown in Table 2:

Table 2 Three series of concentrations of the control

| ng/ml | 25-hydroxyvitamin D (ng/mL) |
|-------|------------------------------|
| L | 6 |
| M | 300 |
| H | 600 |

2. Test results of standard sample and making of standard curve.

dry powder reaction tube has fluorescent reagent -conjugated antibody. The standard curve is made according to test results of 8 series of standard solutions, and a regression equation thereof is as follows:

$$y=0.004553*x+0.1695(r=0.99532).$$

Y is the concentration of the 25-hydroxyvitamin D in sample, X is the intensity of the fluorescent.

3. Analysis of test results of the sample

The fluorescence intensity (fluorescence is used as the label of the antibody dry powder reaction tube) of the test results obtained by the detection card is substituted into the standard curve to calculate the content of 25-hydroxyvitamin D in serum.

4. Accuracy & precision

Low, medium, and high-concentration control samples (L, M, H) to be tested and the fluorescence intensities thereof are substituted into an established standard curve to calculate the concentration of 25-hydroxyvitamin D in the control sample and then calculate the accuracy and precision results of control samples in at least three analysis batches. The acceptance criteria are that the accuracy between the average of measured values and the theoretical value is 85.0%-115.0% and the precision (CV) is ≤15%. Three tests with three concentrations (L, M and H) show that the accuracy is 90.5%-110.5% and the between-run precision (CV) is 1.40%-9.30%, conforming to the requirements.

**Embodiment 2: Influence of combination of different acids and NaCO$_3$ on self-mixing effect**

When a dissociating agent is added to an antibody dry powder reaction tube, citric acid in the dissociating agent reacts with NaCOs in the antibody dry powder reaction tube, resulting in the self-mixing effect. In the embodiment, a detection kit for 25-hydroxyvitamin D is prepared according to the method provided in Embodiment 1, where strong acids in the dissociating agent are different acids as described in Table 1, samples to be tested are serum samples and subjected to liquid chromatography and tandem mass spectrometry detection (detection conditions: mobile phase A: 0.1% formic acid solution; mobile phase B: 0.1% methyl formate; atmospheric pressure chemical ion source (APCI) and multi-reaction monitoring scanning mode (MRM) are used for mass spectrometry), where the content of 25-hydroxyvitamin D is 5.04 ng/mL, and the reaction is carried out for 10 min at ambient temperature. The influence of reaction of different acids with NaCOs on the self-mixing effect is investigated, with the investigation results shown in Table 2. Except that the label is a fluorescent label, others are similar to Embodiment 1, and the test results are calculated by the established standard curve.

Table 2 Influence of reaction of different acids with NaCO$_3$ on self-mixing effect

| Strong acid | Mixing duration | State | 25-hydroxyvitamin D (ng/mL) |
|---|---|---|---|
| Citric acid | More than 10 min | The mixing effect is steady and constant | 5.03 |
| Hydrochloric acid | 8 min | The mixing effect is unsteady, strong at a middle position and weak all around | 4.58 |
| Sulfuric acid | 6 min | The mixing effect is uneven, strong at a middle position and weak all around | 4.01 |
| Nitric acid | 7 min | The mixing effect is unsteady, strong at a middle position and weak all around | 4.33 |
| Control (without any acid) | / | No mixing | 2.17 |

It can be seen from Table 2 that when different strong acids are used to react with NaCOs, the mixing effects are completely different; and the citric acid can react with NaCOs for a long time, thereby providing uninterrupted self-mixing. At the same time, the citric acid also has a good dissociation effect. The citric acid is used as a strong acid to prepare a dissociating agent, such that the test results of 25-hydroxyvitamin D are more accurate.

Embodiment 3 Influence of combination of guanidinium salt and citric acid on test results

In the embodiment, a detection kit for 25-hydroxyvitamin D (labeled with fluorescein) is prepared according to the method provided in Embodiment 1; components of the dissociating agent, having a protein destruction effect, are screened and optimized; components as shown in Table 3 are respectively used to prepare the dissociating agent, and samples to be tested are controls (L); and the influence of different dissociating agents on the test results is investigated, with the investigation results shown in Table 3.

Table 3 Influence of different protein destruction components on test results (the concentrations of the components are the same as those in Embodiment 1)

| Strongly destructive protein component | Test result (fluorescence intensity) | 25-hydroxyvitamin D (ng/mL) |
|---|---|---|
| Guanidine hydrochloride | 1122 | 5.28 |
| Citric acid | 1140 | 5.36 |
| Guanidine hydrochloride + citric acid | 1283 | 6.01 |
| Guanidiniumisothiocyanate | 1111 | 5.23 |
| Acetic acid | 1188 | 5.58 |
| Guanidiniumisothiocyanate + acetic acid | 1138 | 5.35 |

It can be seen from Table 3 that when guanidine hydrochloride or citric acid is used separately or other components with protein destruction ability are used, the dissociation effect may be insufficient, which leads to the deviation of the test results. However, when guanidine hydrochloride and citric acid are combined, the dissociation effect is optimal and the test results of the content of the 25-hydroxyvitamin D are the most accurate. Therefore, the protein destruction component in the dissociating agent is preferably the combination of guanidinium salt and citric acid.

**Embodiment 4 Influence of combination of different surfactants on test results**

A surfactant in a dissociating agent can crack a cell membrane in a sample, such that dissociated free vitamin D can be fully mixed with a reaction substance, and the dissociation time is relatively short. In the embodiment, a detection kit for 25-hydroxyvitamin D (labeled with fluorescein) is prepared according to the method provided in Embodiment 1; components of the surfactants in the dissociating agent are screened and optimized; components as shown in Table 4 are respectively used to prepare the dissociating agent, and samples to be tested are serum samples and subjected to liquid chromatography and tandem mass spectrometry detection, where the content of the 25-hydroxyvitamin D is 10.01 ng/mL, the influence of different surfactants on the test results is investigated, with the investigation results shown in Table 4.

Table 4 Influence of components of different surfactants on test results (the concentrations of the components are the same as those in Embodiment 1)

| Components of surfactants | 25-hydroxyvitamin D (ng/mL) |
|---|---|
| Triton X-100 | 8.28 |
| Surfactant S9 | 8.36 |
| Surfactant S5 | 10.98 |
| Surfactant S19 | 8.23 |
| Surfactant S5 + surfactant S19 | 10.58 |
| Triton X-100 + surfactant S9 | 9.99 |
| Control (no any surfactant) | 7.82 |

It can be seen from Table 4 that when triton X-100 or surfactant S9 is used separately or components of other surfactants are used, non-specific reaction occurs between the dissociated free vitamin D and a subsequent vitamin D antibody, which leads to the test results being high and then low. However, when triton X-100 and surfactant S9 are combined, reaction of the dissociated free vitamin D and the subsequent vitamin D antibody is more specific. The dissociation effect is optimal and the test results of the content of the 25-hydroxyvitamin D are the most accurate. Therefore, the surfactant in the dissociating agent is preferably the combination of triton X-100 and surfactant S9.

**Embodiment 5 Influence of different detergents on test results**

A detergent in a dissociating agent has the function to destroy proteins and lyse cells and can help to fully dissolve substances in an antibody dry powder reaction tube. In the embodiment, a detection kit for 25-hydroxyvitamin D (labeled with fluorescein) is prepared according to the method provided in Embodiment 1; components of the dissociating agent are screened and optimized; components as shown in Table 5 are respectively used to prepare the dissociating agent, and samples to be tested are controls (L); and the influence of different detergents on the test results is investigated, with the investigation results shown in Table 5.

Table 5 Influence of components of different detergents on test results (the concentrations of the components are the same as those in Embodiment 1)

| Components of detergents | 25-hydroxyvitamin D (ng/mL) |
| --- | --- |
| Sodium dodecyl sulfate | 6.0 |
| Stearic acid | 5.36 |
| Sodium dodecyl benzene sulfonate | 5.98 |
| Fatty glyceride | 5.23 |
| Sorbitan fatty acid ester | 5.58 |
| Polysorbate | 5.25 |

It can be seen from Table 5 that compared with other detergents, sodium dodecyl sulfate can help to fully dissolve substances in the antibody dry powder reaction tube when used as the detergent, such that the test results of the content of the 25-hydroxyvitamin D are the most accurate.

**Embodiment 6 Influence of different metal complexing agents on test results**

A metal complexing agent in a dissociating agent can aggregate and precipitate the denatured vitamin D binding protein to prevent the dissociated 25-hydroxyvitamin D from binding with the protein again. This increases an amount of dissociated vitamin D, reduces the dissociation time and increases the accuracy. In the embodiment, a detection kit for 25-hydroxyvitamin D (labeled with fluorescein) is prepared according to the method provided in Embodiment 1; components of the metal complexing agent in the dissociating agent are screened and optimized; components as shown in Table 6 are respectively used to prepare the metal complexing agent, and samples to be tested are controls (L). In addition, a negative serum is tested as a blank sample; and the influence of different metal complexing agents on the test results is investigated, with the investigation results shown in Table 6.

Table 6 Influence of components of different metal complexing agents on test results (the concentrations of the components are the same as those in Embodiment 1)

| Components of metal complexing agents | Blank sample | 25-hydroxyvitamin D (ng/mL) |
| --- | --- | --- |
| Ethylene diamine tetraacetic acid | 0 | 5.99 |
| Diethanol amine | 0.41 | 6.36 |
| Diethylenetriaminepentaacetic acid | 1.15 | 6.98 |
| Sodium ethylenediaminetetramethylene phosphate | 0.25 | 6.23 |
| Sodium alginate | 0.97 | 6.58 |
| Polyacrylic acid | 0.33 | 6.25 |

It can be seen from Table 6 that compared with other metal complexing agents, ethylene diamine tetraacetic acid being used as the metal complexing agent can prevent metal ions from interfering immune reaction, such that the test results of the content of the 25-hydroxyvitamin D are the most accurate. Because the metal ions interfere with immune reaction when other metal complexing agents are used, false positives are prone to appear even if blank samples are tested.

**Embodiment 7 Influence of combination of polyvinyl alcohol and polyvinylpyrrolidone on test results**

An antibody fixative in an antibody dry powder reaction tube can facilitate adhesion of a 25-hydroxyvitamin D antibody to the reaction tube to avoid shedding thereof during transportation. In the embodiment, a detection kit for 25-hydroxyvitamin D (labeled with fluorescein) is prepared according to the method provided in Embodiment 1; components of the antibody fixative in the antibody dry powder reaction tube are screened and optimized; components as shown in Table 7 are respectively used to prepare the antibody fixative, the prepared antibody dry powder reaction tube is placed in a vibrating instrument (with power of 200 w) and vibrated for 1 hour, and samples to be tested are serum samples and subjected to liquid chromatography and tandem mass spectrometry detection, where the content of the 25-hydroxyvitamin D is 8.61 ng/mL, the influence of different antibody fixatives on the test results is investigated, with the investigation results shown in Table 7. In the embodiment, only the antibody adhered to a tube bottom performs reaction during testing.

Table 7 Influence of components of different antibody fixatives on test results (the concentrations of the components are the same as those in Embodiment 1)

| Components of antibody fixatives | 25-hydroxyvitamin D (ng/mL) |
|---|---|
| Polyvinyl alcohol | 8.15 |
| Polyvinylpyrrolidone | 8.06 |
| Carboxymethyl cellulose | 7.28 |
| Polystyrene | 7.23 |
| Carboxymethyl cellulose + styrene | 8.08 |
| Polyvinyl alcohol + polyvinylpyrrolidone | 8.59 |

It can be seen from Table 7 that compared with other antibody fixatives, or compared with polyvinyl alcohol or polyvinylpyrrolidone being used separately, the polyvinyl alcohol and the polyvinylpyrrolidone being used as the antibody fixatives can better adhere the antibody to the reaction tube to avoid shedding thereof during transportation, such that the test results are more accurate. Because a dried antibody has relatively small volume, it is attached to the bottom of the reaction tube under normal circumstances and can be completely used during reaction. When the antibody cannot be adhered to the bottom of the reaction tube, it may fall into other parts of the reaction tube. For example, if the antibody falls onto a sealing aluminum foil of the reaction tube, tearing the aluminum foil during use will lead to the loss of antibody powder. In this case, when the sample or dissociation solution is added, failure of reaction and detection may occur due to the low content of the antibody.

**Embodiment 8 Influence of combination of casein sodium salt and trehalose on test results**

An antibody protective agent in an antibody dry powder reaction tube can effectively protect a colloidal gold-labeled 25-hydroxyvitamin D antibody. In the embodiment, a detection kit for 25-hydroxyvitamin D (labeled with fluorescein) is prepared according to the method provided in Embodiment 1; components of the antibody protective agent in the antibody dry powder reaction tube are screened and optimized; components as shown in Table 8 are respectively used to prepare the antibody protective agent, and samples to be tested are serum samples and subjected to liquid chromatography and tandem mass spectrometry detection, where the content of the 25-hydroxyvitamin D is 7.53 ng/mL, the influence of different antibody protective agents on the test results is investigated, with the investigation results shown in Table 8.

Table 8 Influence of components of different antibody protective agents on test results (the concentrations of the components are the same as those in Embodiment 1)

| Components of antibody protective agents | 25-hydroxyvitamin D (ng/mL) |
|---|---|
| Casein sodium salt | 7.35 |
| Trehalose | 7.36 |

(continued)

| Components of antibody protective agents | 25-hydroxyvitamin D (ng/mL) |
|---|---|
| Sucrose | 7.28 |
| Gelatin | 7.23 |
| Sucrose + gelatin | 7.45 |
| Casein sodium salt + trehalose | 7.50 |
| Control (no any t antibody protective agents) | 6.98 |

It can be seen from Table 7 that compared with other antibody protective agents, or compared with casein sodium salt or trehalose being used separately, the casein sodium salt and the trehalose being used as the antibody protective agents can better protect the colloidal gold-labeled 25-hydroxyvitamin D antibody, such that the test results of the content of the 25-hydroxyvitamin D are the most accurate.

Although fluorescein as the labels in Embodiments 2-8 is used to specifically screen an optimal reagent combination of the present invention, the improvement effect thereof is the same as that of gold particles and latex particles as the labels. This does not the improvement effect of other reagents (specific experimental data are omitted) except that the labels are different. It is easily understood by ordinary persons in the art that labels are only used to visually display results or detect the content and concentration of the reagent used to test an analyte in a sample, without affecting real results. Therefore, actual screening with fluorescein as labels can be quantified, and the use of a colloidal gold-labeled antibody is only a preferred embodiment. When the colloidal gold-labeled antibody is tested, the test result can be read by naked eyes or machine.

All the patents and publications mentioned in the description of the present invention indicate that these are public technologies in the art and can be used by the present invention. All the patents and publications cited herein are listed in the references, just as each publication is specifically referenced separately. The present invention described herein can be realized in the absence of any one element or multiple elements, one restriction or multiple restrictions, where such restriction is not specifically described here. For example, the terms "comprising", "essentially consisting of' and "consisting of' in each embodiment herein may be replaced by the rest 2 terms. The so-called "alan" herein merely means "one", but does not exclude including 2 or more instead of including only one. The terms and expressions which have been employed herein are descriptive rather than restrictive, and there is no intention to suggest that these terms and expressions in this description exclude any equivalents, but it is to be understood that any appropriate changes or modifications can be made within the scope of the present invention and appended claims. It can be understood that the embodiments described in the present invention are some preferred embodiments and features. A person skilled in the art can make some modifications and changes according to the essence of the description of the present invention. These modifications and changes are also considered to fall within the scope of the present invention and the scope limited by independent claims and dependent claims.

## Claims

1. A kit for detecting 25-hydroxyvitamin D in a sample, comprising: a dissociating agent and a dry powdered reagent including a label-conjugated anti-25-hydroxyvitamin D antibody; wherein the dissociating agent comprises a protein decomposer, a surfactant, a detergent, and a metal complexing agent.

2. The kit according to claim 1, wherein the protein decomposer comprises guanidinium salt and strong acid.

3. The kit according to one of claims 1-2, wherein the surfactant comprises triton X-100 and a surfactant S9.

4. The kit according to one of claims 1-3, wherein the detergent comprises sodium dodecyl sulfate.

5. The kit according to one of claims 1-4, wherein the metal complexing agent comprises ethylene diamine tetraacetic acid.

6. The kit according to claim 5, wherein the strong acid is citric acid, and the guanidinium salt is guanidine hydrochloride.

7. The kit according to claim 6, wherein each 1 L of the dissociating agent comprises 1-20 g of guanidine hydrochloride, 1-20 mL of triton X-100, 1-20 g of sodium dodecyl sulfate, 1-20 g of ethylene diamine tetraacetic acid, 50-400 g of citric acid, and 1-20 g of surfactant S9; and the remainder thereof is water.

8. The kit according to one of claims 1-7, wherein the dry powdered reagent further comprises an antibody protective agent, an antibody fixative, and sodium carbonate; and the antibody fixative comprises polyvinyl alcohol and polyvinylpyrrolidone.

9. The kit according to claim 8, wherein the antibody protective agent further comprises casein sodium salt and trehalose.

10. The kit according to one of claims 1-9, wherein the label comprises gold particles, latex particles, a water-soluble dye or a fluorescent substance.

11. The kit according to claim 1, wherein the powdered reagent comprises 0.8-8 $\mu$g of a colloidal gold-conjugated labeled murine anti-25-hydroxyvitamin D monoclonal antibody, 0.008-0.8 $\mu$g of casein sodium salt, 0.008-0.8 $\mu$g of polyvinyl alcohol, 0.008-0.8 $\mu$g of polyvinylpyrrolidone, 400-4000 $\mu$g of sodium carbonate, and 0.8-8 $\mu$g of trehalose.

12. The kit according to claim 1, wherein the dissociating agent is encapsulated in a container, and the powdered reagent is immobilized in a reaction tube.

13. The kit according to one of claims 1-12, wherein the kit further comprises an immunochromatographic test strip, the test strip comprises a testing area and a control area located downstream of the testing area, and a 25-hydroxyvitamin antigen is immobilized on the testing area.

14. The kit according to claim 13, wherein a control line comprises a colloidal gold-labeled murine IgG antibody and 0.005%-0.5% Evans blue, and the Evans blue covers the colloidal gold-labeled murine IgG antibody.

15. The kit according to claim 14, wherein the colloidal gold-labeled murine IgG antibody is immobilized on the testing area and the Evans blue is capable to flow with the liquid.

FIG. 1

Vitamin D

Control line ········ C

Test line ········ T

Reaction area

Sample
application area

Shell

FIG. 2

1drop(approximately 40µl)

3drops(approximately 120µl)

squeeze the bottom 5-10 times

mix well and wait for 5-10min

3 drops of the mixture

Read results at 10-15min

FIG. 3

C

T

10ng

C

T

30ng

C

T

100ng

C

T

C

T

INVALID

FIG. 4

# EUROPEAN SEARCH REPORT

**Application Number**

EP 23 19 7774

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | IN 2448 DEL 2014 A1 (MAHAJAN L) 14 August 2015 (2015-08-14) | 1-13 | INV. G01N33/558 |
| A | * claims 1-6 * | 14,15 | G01N33/82 |
| Y | US 2014/370616 A1 (GUPTA RAJAN [CA] ET AL) 18 December 2014 (2014-12-18) | 1-13 | |
| A | * paragraph [0027]; claims 2-3,18-19 * | 14,15 | |
| Y | CN 110 488 008 A (BEIJING SAVANT BIOTECHNOLOGY CO LTD) 22 November 2019 (2019-11-22) | 1-13 | |
| A | * claims 1-6 * | 14,15 | |
| Y | CN 113 884 687 A (NANJING LETSWIN BIOTECHNOLOGY CO LTD) 4 January 2022 (2022-01-04) | 1-13 | |
| A | * claims 1-3 * | 14,15 | |
| Y | CN 113 495 159 A (ZHENGZHOU DANUO BIOTECH CO LTD) 12 October 2021 (2021-10-12) | 1-13 | |
| A | * paragraph [0032]; claims 1-8 * | 14,15 | TECHNICAL FIELDS SEARCHED (IPC) |
| | | | G01N |

The present search report has been drawn up for all claims

| Place of search | Date of completion of the search | Examiner |
|---|---|---|
| The Hague | 15 February 2024 | van der Kooij, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 19 7774

This annex lists the patent family members relating to the patent documents cited in the above-mentioned European search report.
The members are as contained in the European Patent Office EDP file on
The European Patent Office is in no way liable for these particulars which are merely given for the purpose of information.

15-02-2024

| Patent document cited in search report | | | Publication date | Patent family member(s) | | Publication date |
|---|---|---|---|---|---|---|
| IN 2448DEL2014 | A1 | | 14-08-2015 | ------------------------------------ | | |
| US 2014370616 | A1 | | 18-12-2014 | US 2014370616 A1 | | 18-12-2014 |
| | | | | WO 2012129650 A1 | | 04-10-2012 |
| CN 110488008 | A | | 22-11-2019 | NONE | | |
| CN 113884687 | A | | 04-01-2022 | NONE | | |
| CN 113495159 | A | | 12-10-2021 | NONE | | |

EPO FORM P0459

For more details about this annex : see Official Journal of the European Patent Office, No. 12/82

**EP 4 509 837 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2023110449482
- CN 115166266 A
- CN 115856326 A
- CN 115932289 A
- US 4857453 A
- US 5073484 A
- US 5119831 A
- US 5185127 A
- US 5275785 A
- US 5416000 A
- US 5504013 A
- US 5602040 A
- US 5622871 A
- US 5654162 A
- US 5656503 A
- US 5686315 A
- US 5766961 A
- US 5770460 A
- US 5916815 A
- US 5976895 A
- US 6248598 B
- US 6140136 A
- US 6187269 B
- US 6187598 B
- US 6228660 B
- US 6235241 B
- US 6306642 B
- US 6352862 B
- US 6372515 B
- US 6379620 B
- US 6403383 B